# EUROPEAN PATENT APPLICATION

(11) **EP 2 599 479 A1**
(43) Date of publication of application: **05.06.2013**
(21) Application number: 11009474.5
(22) Date of filing: 30.11.2011
(51) Int. Cl.: A61K 31/13, A61K 31/19, A61K 31/27, A61K 31/445, A61K 31/473, A61K 31/55, A61P 25/28, A61K 9/14

(54) **4-phenylbutyric acid for the treatment of alzheimer's disease**

(71) Applicant: Lunamed AG, 7000 Chur (CH)
(72) Inventor: The designation of the inventor has not yet been filed
(74) Representative: Peters, Hajo

(57) **Abstract**

The present invention relates to the therapeutic use of 4-phenylbutyric acid (4-PB) in the treatment of Alzheimer's disease. Particularly, the present invention provides 4-PB at low daily dosis for use in the treatment of Alzheimer's disease, pharmaceutical compositions and kits comprising 4-PB at low dosis for the treatment of Alzheimer's disease, and methods for treatment of Alzheimer's disease involving the administration of 4-PB at low daily dosis.

## Description

### Field of the invention

The present invention relates to the therapeutic use of 4-phenylbutyric acid (4-PB) in the treatment of Alzheimer's disease. Particularly, the present invention provides 4-PB at low daily dosis for use in the treatment of Alzheimer's disease, pharmaceutical compositions and kits comprising 4-PB at low dosis for the treatment of Alzheimer's disease, and methods for treatment of Alzheimer's disease involving the administration of 4-PB at low daily dosis.

### Background of the invention

Alzheimer's disease (also called primary degenerative dementia of the Alzheimer's type, Alzheimer's or short AD) is an age-related, so far non-reversible neurodegenerative disorder that develops over a period of years and is the most common form of dementia among older people.

Characteristic is a progressively deterioration of the cognitive performance which usually is accompanied by a decrease in daily activity, behaviour and personality changes and neuropsychological symptoms. Ultimately, Alzheimer's disease leads to a sever loss of mental functions. There are three major changes occurring within the brain years before the first clinical symptoms manifest: Formation of amyloid plaques, which are made up of erroneously folded beta-amyloid peptides, and neurofibrillary tangles (NFTs), which are abnormal collections of a protein called tau, and loss of connections between neurons responsible for memory and learning.

Currently there are no medicines that can slow the progression of Alzheimer's disease. However, four medications have been approved by the United States Food and Drug Administration (FDA) for the treatment of Alzheimer's symptoms. Donepezil, rivastigmine and galantamine are prescribed to treat mild to moderate symptoms of Alzheimer's, while the newest medication memantine is prescribed to treat moderate to severe symptoms. However, these four medications will not stop or reverse Alzheimer's. Accordingly, there is a need for alternative treatments of Alzheimer's disease.

4-Phenylbutyric acid (4-PB) is a low molecular weight aromatic carboxylic acid. 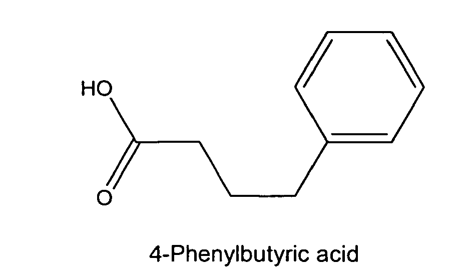 "4-Phenylbutyric acid" (abbreviated as "4-PB") is defined herein as encompassing not only 4-Phenybutyric acid, as free acid but also its derivatives, and physiologically/ pharmaceutically acceptable salts thereof. Especially, "4-Phenylbutyric acid" or "4-PB" is also defined as its free acid, but also as being in the form of a pharmaceutically acceptable salt, co-crystal, polymorph, hydrate, solvate or pro-drug of 4-phenylbutyric acid. Most preferably, "4-Phenylbutyric acid" or "4-PB" is either the free acid or a pharmaceutically acceptable salt of 4-PB, such as its sodium salt. This definition is to be applied in all cases within this description or the attached claims in which the expression "4-Phenylbutyric acid" or "4-PB" is used unless being more precisely defined as e.g. 4-Phenylbutyric acid as free acid or sodium salt of 4-PB. Thus, 4-Phenylbutyric acid (or 4-PB) as free acid is exactly the compound whose structure is shown above, while a sodium salt of 4-PB is exactly the sodium salt of the 4-Phenylbutyric acid, whose structure is shown above.

The 4-phenylbutyric acid sodium salt (sodium 4-phenylbutyrate) and its use for the treatment of various clinical conditions and diseases such as benign prostate hyperplasy, cancer, cystic fibrosis, HIV, kidney and liver failure, thalasemia and urea cycle disorders are known. 4-PB has also been suggested being potentially useful in the treatment of Alzheimer's disease.

However no proof of for this potential activity has been presented so far and the currently provided high dosage treatments and forms seem to have been not to be very effective in the treatment of Alzheimer's disease. Therefore, there is a need for improved treatment forms of Alzheimer's disease.

### Summary of the invention

The present inventors have surprisingly found that 4-PB when administered at low dosis ameliorates and effectively treats the symptoms associated with Alzheimer's disease.

Based on the above findings, the present inventors provide the following aspects which summarize the present invention.

One aspect of the invention relates to 4-phenylbutyric acid (4-PB) for use in the treatment of Alzheimer's disease, wherein 4-PB is administered to a subject in need thereof in a daily amount of at most about 750 mg.

Another aspect of the invention relates to a pharmaceutical composition for use in the treatment of Alzheimer' disease, comprising 4-phenylbutyric acid in an amount of at most about 750mg and one or more pharmaceutically acceptable carriers, diluents and/or excipients.

Another aspect of the invention relates to a kit for use in the treatment of Alzheimer's disease, comprising at least one container which contains a pharmaceutical formulation comprising 4-phenylbutyric acid in an amount of at most about 750 mg, and, optionally, at least a second container which contains a pharmaceutical formulation comprising one or more amino acids and/or at least one other therapeutic agent effective in the treatment of Alzheimer's disease.

Another aspect of the invention relates to the use of phenylbutyric acid in the manufacture of a medicament for the treatment of Alzheimer's disease, wherein 4-PB is administered to a subject in need thereof in a daily amount of at most 750 mg.

Another aspect of the invention relates to methods for the treatment of Alzheimer's disease, comprising administering to a subject in need thereof 4-phenylbutyric acid in a daily amount of at most 750 mg.

Having summarized the various aspects of the present invention, the invention is now described in more detail. It is intended that each and every embodiment described hereafter may form part of any one of the above aspects. Moreover, it is explicitly intended that the teaching of this invention covers any combination of these embodiments.

### Detailed description of the invention

The present invention provides 4-phenylbutyric acid (4-PB) for use in the treatment of Alzheimer's disease. According to the invention, 4-PB is administered to a subject in need thereof in a daily amount of at most about 750 mg.

According to the invention, a subject or patient is an organism that exhibits the properties or symptoms associated with Alzheimer's disease. The subject or patient is typically a vertebrate, in particular a mammal. According to some embodiments, the subject or patient is a human (Homo sapiens). The terms "subject" and "patient" may be used interchangeably. According to some embodiments, the daily amount of 4-PB to be administered may be at most about 700 mg, at most about 650 mg, at most about 600 mg, at most about 550 mg, at most about 500 mg, at most about 450 mg, at most about 400 mg, at most about 350 mg, at most about 300 mg, at most about 250 mg, at most about 200 mg, at most about 150 mg, at most about 100 mg, at most about 75 mg, at most about 50 mg or at most about 25 mg.

According to some embodiments, the daily amount of 4-PB to be administered is at most about 100 mg

According to some embodiments, the daily amount of 4-PB to be administered is at most about 250 mg.

According to some embodiments, the daily amount of 4-PB to be administered is at most about 500 mg.

According to some embodiments, the daily amount of 4-PB to be administered is at least about 25 mg, at least about 50 mg, at least about 75 mg, at least about 100 mg, at least about 150 mg, at least about 200 mg, at least about 250 mg, at least about 300 mg, at least about 350, at least about 400 mg, at least about 450 mg, at least about 500 mg, at least about 550 mg, at least about 600 mg, at least about 650 mg or at least about 700 mg.

According to some embodiments, the daily amount of 4-PB to be administered is at least about 25 mg.

According to some embodiments, the daily amount of 4-PB to be administered is at least about 50 mg.

According to some embodiments, the daily amount of 4-PB to be administered is at least about 75 mg.

According to some embodiments, the daily amount of 4-PB to be administered is at least about 100 mg.

According to some embodiments, the daily amount of 4-PB to be administered is at least about 250 mg.

According to some embodiments, the daily amount of 4-PB to be administered is at least about 500 mg.

According to some embodiments, the daily amount of 4-PB to be administered is in the range from about 25 mg to about 750 mg, from about 50 mg to about 750 mg, from about 75 mg to about 750, about 100 mg to about 750 mg, from about 150 mg to about 750 mg, from about 200 mg to about 750 mg, from about 250 mg to about 750 mg, from about 300 mg to about 750 mg, from about 350 mg to about 750 mg, from about 400 mg to about 750 mg, from about 450 mg to about 750 mg, from about 500 mg to about 750 mg, from about 550 mg to about 750 mg, from about 600 mg to about 750 mg, from about 650 to about 750 mg or from about 700 mg to about 750 mg.

According to some embodiments, the daily amount of 4-PB to be administered is in the range from about 25 mg to about 500 mg, from about 50 mg to about 500 mg, from about 75 mg to about 500 mg, from about 100 to about 500 mg, from about 150 to about 500 mg, from about 200 mg to about 500 mg, from about 250 mg to about 500 mg, from about 300 mg to about 500 mg, from about 350 mg to about 500 mg, from about 400 mg to about 500 mg or from about 450 to about 500 mg.

According to some embodiments, the daily amount of 4-PB to be administered is in the range from about 25 mg to about 250 mg, from about 50 mg to about 250 mg, from about 75 mg to about 250 mg, from about 100 to about 250 mg, from about 125 mg to about 250 mg, from about 150 to about 250 mg, from about 175 mg to about 250 mg or from about 200 mg to about 250 mg.

According to some embodiments, the daily amount of 4-PB to be administered is in the range from about 25 mg to about 100 mg, from about 50 mg to about 100 mg, from about 55 mg to about 100 mg, from about 60 mg to about 100 mg, from about 65 mg to about 100 mg, from about 70 mg to about 100 mg, from about 75 mg to about 100 mg, from about 80 mg to about 100 mg, from about 85 mg to about 100 mg, from about 90 mg to about 100 mg or from about 95 to about 100 mg.

According to some embodiments, 4-PB is administered one or more times a day, such as, e.g., two times, three times, four times or five times. According to some embodiments, 4-PB is administered once a day. According to some embodiments, 4-PB is administered twice a day. According to some embodiments, 4-PB is administered once in the morning and once in the evening.

According to some embodiments, 4-PB is administered two times or more a day, such as, e.g., three times, four times or five times, wherein the administrations are 2 to 14 hours apart, such as, e.g., 4, 6, 8, 10 or 12 hours apart. According to specific embodiments, 4-PB is administered twice a day, wherein the administrations are 8 to 12 hours apart.

According to some embodiments, 4-PB is administered in form of a pharmaceutically acceptable salt, co-crystal, polymorph, hydrate, solvate or pro-drug. The phrase "pharmaceutically acceptable", as used herein, means those salts, co-crystals, polymorphs, hydrates, solvates and pro-drugs of 4-PB that are safe in terms of toxicity, irritation, allergic response, or other problem or compliance, and effective for therapeutic use in mammals, in particular humans.

Pharmaceutically acceptable salts of 4-PB are known in the art and include salts derived from inorganic or organic acids. A pharmaceutically acceptable salt of 4-PB suitable for use in the present invention can be an alkali metal salt, an earth alkali metal salt, an ammonium salt, a substituted ammonium salt, or an acid addition salt.

The alkali metal salts can be prepared from the free acid by means well known to those of skill in the art (e.g., reaction of the free carboxylic acid with an alkali metal hydroxide or alkoxide in an appropriate solvent). The acid addition salts of 4-PB suitable can be generated from the free-base forms of the compounds by reaction of the latter with one equivalent of a suitable, non-toxic, pharmaceutically-acceptable acid, followed by evaporation of the solvent employed for the reaction and recrystallization of the salt, as required. Suitable acids for forming acid addition salts of 4-PB include, but are not limited to, acetic, benzoic, benzenesulfonic, tartaric, hydrobromic, hydrochloric, citric, fumaric, gluconic, glucuronic, glutamic, lactic, malic, maleic, methanesulfonic, palmoic, salicylic, stearic, succinic, sulfuric, and tartaric acids. The class of acids suitable for formation of nontoxic, pharmaceutically-acceptable salts is well known to the persons skilled in the art, and are described, for example in Stahl, P.H., at al., "Handbook of Pharmaceutical Salts", Wiley-VCH, Weinheim: Germany (2002). The free base of 4-PB, if required, can be recovered from an acid addition salt thereof by reaction of the salt with a water solution of the salt with a suitable base such as sodium carbonate, sodium hydroxide, and the like.

The salts can be prepared in situ during the final isolation and purification of the compounds of the present invention or separately by reacting a free base function with a suitable organic acid. Representative acid addition salts include, but are not limited to acetate, adipate, alginate, citrate, aspartate, benzoate, benzenesulfonate, bisulfate, butyrate, camphorate, camphorsufonate, digluconate, glycerophosphate, hemisulfate, heptanoate, hexanoate, fumarate, hydrochloride, hydrobromide, hydroiodide, 2-hydroxyethansulfonate (isethionate), lactate, maleate, methanesulfonate, nicotinate, 2-naphthalenesulfonate, oxalate, pamoate, pectinate, persulfate, 3-phenylpropionate, picrate, pivalate, propionate, succinate, tartrate, thiocyanate, phosphate, glutamate, bicarbonate, p-toluenesulfonate and undecanoate. Also, the basic nitrogen-containing groups can be quaternized with such agents as lower alkyl halides such as methyl, ethyl, propyl, and butyl chlorides, bromides and iodides; dialkyl sulfates like dimethyl, diethyl, dibutyl and diamyl sulfates; long chain halides such as decyl, lauryl, myristyl and stearyl chlorides, bromides and iodides; arylalkyl halides like benzyl and phenethyl bromides and others. Water or oil-soluble or dispersible products are thereby obtained. Examples of acids which can be employed to form pharmaceutically acceptable acid addition salts include such inorganic acids as hydrochloric acid, hydrobromic acid, sulphuric acid and phosphoric acid and such organic acids as oxalic acid, maleic acid, succinic acid and citric acid.

Basic addition salts can be prepared in situ during the final isolation and purification of compounds of this invention by reacting a carboxylic acid-containing moiety with a suitable base such as the hydroxide, carbonate or bicarbonate of a pharmaceutically acceptable metal cation or with ammonia or an organic primary, secondary or tertiary amine. Pharmaceutically acceptable salts include, but are not limited to, cations based on alkali metals or alkaline earth metals such as lithium, sodium, potassium, calcium, magnesium and aluminum salts and the like and nontoxic quaternary ammonia and amine cations including ammonium, tetramethylammoniurn, tetraethylammonium, methylamine, dimethylamine, trimethylarnine, triethylamine, diethylamine, ethylamine and the like. Other representative organic amines useful for the formation of base addition salts include ethylenediamine, ethanolamine, diethanolamine, piperidine, piperazine and the like. Salts of 4-PB also include phosphate, tris and acetate.

Pharmaceutical acceptably salts may be also obtained using standard procedures well known in the art, for example by reacting a sufficiently basic compound such as an amine with a suitable acid affording a physiologically acceptable anion. Alkali metal (for example, sodium, potassium or lithium) or alkaline earth metal (for example calcium or magnesium) salts of carboxylic acids can also be made.

According to some embodiments, the 4-PB salt is an alkali metal salt selected from the group of lithium, sodium, potassium, calcium, magnesium, aluminium and cesium.

According to some embodiments, the 4-PB salt is the sodium salt, sodium 4-phenylbutyrate.

A co-crysal of 4-PB, as referred to herein, is a crystalline material comprising two or more compounds at ambient temperature (20 to 25°C, in particular 20°C), of which at least two are held together by weak interaction, wherein at least one of the compounds is a co-crystal former. Weak interaction is being defined as an interaction, which is neither ionic nor covalent and includes for example: hydrogen bonds and van der Waals forces. Solvates or salts of phenyl butyric acid and an organic compound that do not further comprise a co-crystal former are not co-crystals according to the present invention. The co-crystals may however, include one or more solvate molecules in the crystalline lattice. Just for the sake of clarity the distinction between crystalline salt and a co-crystal has to be stressed here. An API bound to another compound forming a salt by means of ionic interaction can be considered as one "compound" according to the invention, but it cannot be considered as two compounds by itself. See also Zaworotko (Zaworotko, Crystal Growth & Design, Vol. 7, No. 1, 2007, 4-9) which gives a definition of co-crystal which is in line with the definition given above.

Polymorphs of 4-PB, as referred to herein, are crystalline forms of 4-PB having the same chemical composition but different spatial arrangements of the molecules, atoms, and/or ions forming the crystal. "Polymorphs" in general are described in "Polymorphism in Pharmaceutical Solids", Harry G. Brittain (ed.) Drugs and the Pharmaceutical Sciences 192 (1999).

A solvate of 4-PB, as referred to herein, is a physical association of 4-PB with one or more solvent molecules, whether organic or inorganic. This physical association includes hydrogen bonding. In certain instances the solvate will be capable of isolation, for example when one or more solvent molecules are incorporated in the crystal lattice of the crystalline solid. The solvate may comprise either a stoichiometric or nonstoichiometric amount of the solvent molecules. For example, a solvate with a nonstoichiometric amount of solvent molecules may result from partial loss of solvent from the solvate. "Solvate" encompasses both solution-phase and isolable solvates. Exemplary solvates include hydrates, ethanolates, methanolates, isopropanolates and the like. Methods of solvation are generally known in the art.

A hydrate of 4-PB, as referred to herein, is a substance containing 4-PB and water. It may be a crystalline salt with one or more water molecules in the crystalline structure or also amorphous containing water molecules.

A prodrug of 4-PB is a compound that is transformed in vivo to 4-PB, for example, by hydrolysis. Prodrug design is discussed generally in Hardma et al. (Eds.), Goodman and Gilman's The Pharmacological Basis of Therapeutics, 9th ed., pp. 11-16 (1996). Another discussion is also provided by Higuchi, et al., in Prodrugs as Novel Delivery Systems. Vol. 14. ASCD Symposium Series, and in Roche (ed.), Bioreversible Carriers in Drug Design. American Pharmaceutical Association and Pergamon Press (1987). Typically, administration of a drug is followed by elimination from the body or some biotransformation whereby the biological activity of the drug is reduced or eliminated. Alternatively, a biotransformation process can lead to a metabolic by-product that is more or equally active compared to the drug initially administered. Increased understanding of these biotransformation processes permits the design of so-called "prodrugs," which, following a biotransformation, become more physiologically active in their altered state. Prodrugs, therefore, as used within the scope of the present disclosure, encompass compounds that are converted by some means to pharmacologically active metabolites.

According to some embodiments, 4-PB is administered in combination with one or more amino acids (such as, e.g. two, three, four, five, six, seven, eight, nine or ten amino acids). The one or more amino acids may be proteinogenic and/or non-proteinogenic. Proteinogenic amino acids, as referred to herein, are those amino acids that can be found in proteins and require cellular machinery coded for in the genetic code of any organism for their isolated production. Proteinogenic amino acids thus include the 22 standard amino acids. Non-proteinogenic amino acids, as referred to herein, are those amino acids which are either not found in proteins, or are not produced directly and in isolation by standard cellular machinery.

According to some embodiments, the one or more amino acids are L-amino acids or D-amino acids, or a combination thereof.

According to specific embodiments, the one or more amino acids is/are in the D-configuration.

According to some embodiments, the one or more amino acids is/are selected from nonpolar, polar, acidic or basic amino acids.

According to some embodiments, the one or more amino acids is/are selected from aliphatic, sulfur-containing, aromatic or neutral amino acids.

According to some embodiments, the one or more amino acids is/are selected from alanine, valine, leucine, isoleucine, proline, methionine, phenylalanine and tryptophane.

According to some embodiments, the one or more amino acids is/are selected from serine, threonine, asparagine, glutamine, cysteine and tyrosine.

According to some embodiments, the one or more amino acids is/are selected from aspartic acid and glutamic acid.

According to some embodiments, the one or more amino acids is/are selected from lysine, arginine and histidine.

According to some embodiments, the one or more amino acids is/are selected from alanine, valine, Leucine and isoleucine.

According to some embodiments, the one or more amino acids is/are selected from cysteine and methionine.

According to some embodiments, the one or more amino acid is/are selected from phenylalanine, tyrosine and tryptophane.

According to some embodiments, the one or more amino acid is/are selected from serine, threonine, asparagine and glutamic acid.

According to some embodiments, the one or more amino acid is/are selected from selenocysteine, selenomethionine, norleucine, norvaline, ornithine, penicillamine, hydroxyproline, homoserine, homophenylalanine, homoleucine, homonorleucine and homoarginine,

According to some embodiments, the one or more amino acid is selected from alanine, arginine, aspargine, aspartic acid, cysteine, glutamic acid, glutamine, histidine, Isoleucine, leucine, lysine, methionine, phenylalanine, proline, serine, threonine, tryptophane, tyrosine, valine, selenocysteine, selenomethionine, norleucine, norvaline, ornithine, penicillamine, hydroxyproline, homoserine, homophenylalanine, homonorleucine and homoarginine.

According to some embodiments, the one amino acid is alanine, especially D-alanine.

According to some embodiments, the one or more amino acids is/are not carnitine, homocystein and canavanine, neither in the L- nor in the D-configuration.

According to some embodiments, the amount of the one or more amino acids to be administered in combination with 4-PB is in the range from about 1:10 to about 10:1 w/w of the amount of 4-PB, each or total.

According to some embodiments, the amount of the one or more amino acids to be administered in combination with 4-PB is in the range from about 1:5 to about 5:1 w/w of the amount of 4-PB, each or total.

According to some embodiments, the amount of the one or more amino acids to be administered in combination with 4-PB is in the range from about 1:2 to about 2:1 w/w of the amount of 4-PB, each or total.

According to some embodiments, the amount of the one or more amino acids to be administered in combination with 4-PB is 1:1 w/w of the amount of 4-PB, each or total. According to some embodiments, the 4-PB used according to the invention is administered one or more times a day, such as, e.g., two times, three times, four times or five times.

According to some embodiments, 4-PB is administered by sustained release, preferably sustained release by oral administration.

By sustained release of the active ingredient is to be understood especially a rate of release of the active ingredient during a period of about 6 to 12 or up to 24 hours.

According to some embodiments, 4-PB is administered in combination with at least another treatment of Alzheimer's disease.

According to some embodiments, the treatment of Alzheimer's disease is a therapeutic agent effective in the treatment of Alzheimer's disease.

According to some embodiments, the therapeutic agent effective in the treatment of Alzheimer's disease is a cholinesterase inhibitor. Non-limiting examples of cholinesterase inhibitor are donepezil, galantamine, rivastigmine and tacrine. Other cholinesterase inhibitors are well known to the person skilled in the art.

According to some embodiments, the therapeutic agent effective in the treatment of Alzheimer's disease is a N-methyl D-aspartate (NMDA) antagonist. A non-limiting example of a NMDA antagonist is memantine. Other NMDA antagonists are well known to the person skilled in the art.

According to some embodiments, the therapeutic agent effective in the treatment of Alzheimer's disease is a BACE 1 inhibitor. BACE 1 inhibitors are well known to the person skilled in the art, and are reviewed, for example in Guo, T., and Hobbs, D.W., Curr. Med. Chem., vol. 13, no. 15, 2006, 1811-29, and Evin, G., et al., Recent Pat CNS Drug Discov., vol. 6, no. 2" 2011, 91-106, which are hereby incorporated by reference and form part of the disclosure. Non-limiting examples of BACE 1 inhibitors are KMI-420, KMI-429, KMI-358, KMI-370, TAK-070, and GSK188909.

According to some embodiments, the therapeutic agent effective in the treatment of Alzheimer's disease is selected from donepezil, galantamine, rivastigmine, tacrine, memantine, KMI-420, KMI-429, KMI-358, KMI-370, TAK-070, and GSK188909.

According to specific embodiments, the therapeutic agent effective in the treatment of Alzheimer's disease is donepezil.

According to specific embodiments, the therapeutic agent effective in the treatment of Alzheimer's disease is galantamine.

According to specific embodiments, the therapeutic agent effective in the treatment of Alzheimer's disease is rivastigmine.

According to specific embodiments, the therapeutic agent effective in the treatment of Alzheimer's disease is tacrine.

According to specific embodiments, the therapeutic agent effective in the treatment of Alzheimer's disease is memantine.

According to specific embodiments, the therapeutic agent effective in the treatment of Alzheimer's disease is selected from KMI-420, KMI-429, KMI-358, KMI-370, TAK-070, and GSK188909.

According to some embodiments, 4-PB is administered in combination with at least another treatment of Alzheimer's disease, wherein a salt or co-crystal of 4-PB with a therapeutic agent effective in the treatment of Alzheimer's disease is administered.

For a definition of co-crystal reference is made to the preceding definition given above.

According to some embodiments, the therapeutic agent effective in the treatment of Alzheimer's disease which is administered while forming a salt or co-crystal with 4-PB (as free acid) is selected from donepezil, galantamine, rivastigmine, tacrine, and memantine.

According to very specific embodiments, the therapeutic agent effective in the treatment of Alzheimer's disease which is administered while forming a salt or co-crystal with 4-PB (as free acid) is donepezil.

According to specific embodiments, the therapeutic agent effective in the treatment of Alzheimer's disease which is administered while forming a salt or co-crystal with 4-PB (as free acid) is galantamine.

According to specific embodiments, the therapeutic agent effective in the treatment of Alzheimer's disease which is administered while forming a salt or co-crystal with 4-PB (as free acid) is rivastigmine.

According to specific embodiments, the therapeutic agent effective in the treatment of Alzheimer's disease which is administered while forming a salt or co-crystal with 4-PB (as free acid) is tacrine.

According to specific embodiments, the therapeutic agent effective in the treatment of Alzheimer's disease which is administered while forming a salt or co-crystal with 4-PB (as free acid) is memantine.

The present invention further provides medicaments, pharmaceutical compositions and pharmaceutical formulations for use in the treatment of Alzheimer's disease. According to the invention, the medicaments, pharmaceutical compositions and pharmaceutical formulations comprises 4-PB in an amount of at most about 750 mg and one or more pharmaceutically acceptable carriers, diluents and/or excipients. The terms "medicament", "pharmaceutical composition" and "pharmaceutical formulations" may be used interchangeably.

The medicament or pharmaceutical composition according to the invention can be of any form suitable for the application to humans and/or animals, preferably humans including infants, children and adults and can be produced by standard procedures known to those skilled in the art. The medicament or pharmaceutical composition can be produced by standard procedures known to those skilled in the art, e.g. from the table of contents of "Pharmaceutics: The Science of Dosage Forms", Second Edition, Aulton, M.E. (ED. Churchill Livingstone, Edinburgh (2002); "Encyclopedia of Pharmaceutical Technology", Second Edition, Swarbrick, J. and Boylan J.C. (Eds.), Marcel Dekker, Inc. New York (2002); "Modern Pharmaceutics", Fourth Edition, Banker G.S. and Rhodes C.T. (Eds.) Marcel Dekker, Inc. New York 2002 y "The Theory and Practice of Industrial Pharmacy", Lachman L., Lieberman H. And Kanig J. (Eds.), Lea & Febiger, Philadelphia (1986). The respective descriptions are hereby incorporated by reference and form part of the disclosure. The composition of the medicament may vary depending on the route of administration.

Carriers, diluents and excipients which are suitable for the preparation of a medicament or pharmaceutical composition according to the present invention are well known to those skilled in the art, e.g. from the "Handbook of Pharmaceutical Excipients" Sixth Edition, Raymond C. Rowe, Paul J. Sheskey and Marian E Quinn (Eds.), American Pharmaceutical Association (July 2009), which is hereby incorporated by reference and forms part of the disclosure.

The medicament or pharmaceutical composition of the present invention may for example be administered parentally in combination with conventional injectable liquid carriers, such as water or suitable alcohols. Conventional pharmaceutical excipients for injection, such as stabilizing agents, solubilizing agents, and buffers, may be included in such injectable compositions. These medicaments or pharmaceutical formulation may for example be injected intramuscularly, intraperitoneally, or intravenously.

Medicaments or pharmaceutical composition according to the present invention may also be formulated into orally administrable compositions containing one or more pharmaceutically acceptable carriers, diluents and/or excipients, in solid or liquid form. These compositions may contain conventional ingredients such as binding agents, fillers, lubricants, and acceptable wetting agents. The compositions may take any convenient form, such as tablets, pellets, granules, capsules, lozenges, aqueous or oily solutions, suspensions, emulsions, or dry powdered forms suitable for reconstitution with water or other suitable liquid medium before use, for immediate or modified or controlled release. The multiparticulate forms, such as pellets or granules, may e.g. be filled into a capsule, compressed into tablets or suspended in a suitable liquid.

Suitable controlled release formulations, materials and methods for their preparation are known from the prior art, e.g. from the table of contents of "Modified-Release Drug Delivery Technology", Rathbone, M.J. Hadgraft, J. and Roberts, M.S. (Eds.), Marcel Dekker, Inc., New York (2002); "Handbook of Pharmaceutical Controlled Release Technology", Wise, D.L. (Ed.), Marcel Dekker, Inc. New York, (2000); "Controlled Drug Delivery", Vol, I, Basic Concepts, Bruck, S.D. (Ed.), CRD Press Inc., Boca Raton (1983) y de Takada, K. and Yoshikawa, H., "Oral Drug Delivery", Encyclopedia of Controlled Drug Delivery, Mathiowitz, E. (Ed.), John Wiley & Sons, Inc., New York (1999), Vol. 2, 728-742; Fix, J., "Oral drug delivery, small intestine and colon", Encyclopedia of Controlled Drug Delivery, Mathiowitz, E. (Ed.), John Wiley & Sons, Inc., New York (1999), Vol. 2, 698-728. The respective descriptions are hereby incorporated by reference and form part of the disclosure.

Medicaments or pharmaceutical composition according to the present invention may also comprise an enteric coating, so that their dissolution is dependent on pH-value. Due to said coating the medicament may pass the stomach undissolved and the respective salts or their respective crystalline form is liberated in the intestinal tract. Preferably the enteric coating is soluble at a pH value of 5 to 7.5. Suitable materials and methods for the preparation are known from the prior art.

The medicament or pharmaceutical composition according to the present invention may be administered as detailed above.

According to some embodiments, the amount of 4-PB in the medicament or pharmaceutical composition is at most about 700 mg, at most about 650 mg, at most about 600 mg, at most about 550 mg, at most about 500 mg, at most about 450 mg, at most about 400 mg, at most about 350 mg, at most about 300 mg, at most about 250 mg, at most about 200 mg, at most about 150 mg, at most about 100 mg, at most about 75 mg, at most about 50 mg or at most about 25 mg.

According to some embodiments, the amount of 4-PB in the medicament or pharmaceutical composition is at most about 100 mg

According to some embodiments, the amount of 4-PB in the medicament or pharmaceutical composition is at most about 250 mg.

According to some embodiments, the amount of 4-PB in the medicament or pharmaceutical composition is at most about 500 mg.

According to some embodiments, the amount of 4-PB in the medicament or pharmaceutical composition is at least about 25 mg, at least about 50 mg, at least about 75 mg, at least about 100 mg, at least about 150 mg, at least about 200 mg, at least about 250 mg, at least about 300 mg, at least about 350, at least about 400 mg, at least about 450 mg, at least about 500 mg, at least about 550 mg, at least about 600 mg, at least about 650 mg or at least about 700 mg.

According to some embodiments, the amount of 4-PB in the medicament or pharmaceutical composition is at least about 25 mg.

According to some embodiments, the amount of 4-PB in the medicament or pharmaceutical composition is at least about 50 mg.

According to some embodiments, the amount of 4-PB in the medicament or pharmaceutical composition is at least about 75 mg.

According to some embodiments, the amount of 4-PB in the medicament or pharmaceutical composition is at least about 100 mg.

According to some embodiments, the amount of 4-PB in the medicament or pharmaceutical composition is at least about 250 mg.

According to some embodiments, the amount of 4-PB in the medicament or pharmaceutical composition is at least about 500 mg.

According to some embodiments, the amount of 4-PB in the medicament or pharmaceutical composition is in the range from about 25 mg to about 750 mg, from about 50 mg to about 750 mg, from about 75 mg to about 750, about 100 mg to about 750 mg, from about 150 mg to about 750 mg, from about 200 mg to about 750 mg, from about 250 mg to about 750 mg, from about 300 mg to about 750 mg, from about 350 mg to about 750 mg, from about 400 mg to about 750 mg, from about 450 mg to about 750 mg, from about 500 mg to about 750 mg, from about 550 mg to about 750 mg, from about 600 mg to about 750 mg, from about 650 to about 750 mg or from about 700 mg to about 750 mg.

According to some embodiments, the amount of 4-PB in the medicament or pharmaceutical composition is in the range from about 25 mg to about 500 mg, from about 50 mg to about 500 mg, from about 75 mg to about 500 mg, from about 100 to about 500 mg, from about 150 to about 500 mg, from about 200 mg to about 500 mg, from about 250 mg to about 500 mg, from about 300 mg to about 500 mg, from about 350 mg to about 500 mg, from about 400 mg to about 500 mg or from about 450 to about 500 mg.

According to some embodiments, the amount of 4-PB in the medicament or pharmaceutical composition is in the range from about 25 mg to about 250 mg, from about 50 mg to about 250 mg, from about 75 mg to about 250 mg, from about 100 to about 250 mg, from about 125 mg to about 250 mg, from about 150 to about 250 mg, from about 175 mg to about 250 mg or from about 200 mg to about 250 mg.

According to some embodiments, the amount of 4-PB in the medicament or pharmaceutical composition is in the range from about 25 mg to about 100 mg, from about 50 mg to about 100 mg, from about 55 mg to about 100 mg, from about 60 mg to about 100 mg, from about 65 mg to about 100 mg, from about 70 mg to about 100 mg, from about 75 mg to about 100 mg, from about 80 mg to about 100 mg, from about 85 mg to about 100 mg, from about 90 mg to about 100 mg or from about 95 to about 100 mg.

According to some embodiments, the medicament or pharmaceutical composition comprises 4-PB in the form of a pharmaceutically acceptable salt, co-crystal, polymorph, hydrate, solvate or pro-drug. For further details on pharmaceutically acceptable salts, co-crystals, polymorphs, hydrates, solvates and pro-drugs of 4-PB reference is made to above description.

According to some embodiments, the 4-PB salt is an alkali metal salt selected from the group of lithium, sodium, potassium, calcium, magnesium, aluminium and cesium.

According to some embodiments, the 4-PB salt is the sodium salt, sodium 4-phenylbutyrate.

According to some embodiments, the medicament or pharmaceutical composition further comprises one or more amino acids (such as, e.g. two, three, four, five, six, seven, eight, nine or ten amino acids).

According to some embodiments, the one or more amino acids is/are L-amino acid(s) or D-amino acid(s), or a combination thereof.

According to specific embodiments, the one or more amino acids is/ are in the D-configuration.

According to some embodiments, the one or more amino acids is/are selected from nonpolar, polar, acidic or basic amino acids.

According to some embodiments, the one or more amino acids is/are selected from aliphatic, sulfur-containing, aromatic or neutral amino acids.

According some embodiments, the one or more amino acids is/are selected from alanine, valine, leucine, isoleucine, proline, methionine, phenylalanine and tryptophane.

According some embodiments, the one or more amino acids is/are selected from serine, threonine, asparagine, glutamine, cysteine and tyrosine.

According some embodiments, the one or more amino acids is/are selected from aspartic acid and glutamic acid.

According some embodiments, the one or more amino acids is/are selected from lysine, arginine and histidine.

According some embodiments, the one or more amino acids is/are selected from alanine, valine, Leucine and isoleucine.

According some embodiments, the one or more amino acids is/are selected from cysteine and methionine.

According some embodiments, the one or more amino acid is/are selected from phenylalanine, tyrosine and tryptophane.

According some embodiments, the one or more amino acid is/are selected from serine, threonine, asparagine and glutamic acid.

According some embodiments, the one or more amino acid is/are selected from selenocysteine, selenomethionine, norleucine, norvaline, ornithine, penicillamine, hydroxyproline, homoserine, homophenylalanine, homoleucine, homonorleucine and homoarginine,

According some embodiments, the one or more amino acid is selected from alanine, arginine, aspargine, aspartic acid, cysteine, glutamic acid, glutamine, histidine, Isoleucine, leucine, lysine, methionine, phenylalanine, proline, serine, threonine, tryptophane, tyrosine, valine, selenocysteine, selenomethionine, norleucine, norvaline, ornithine, penicillamine, hydroxyproline, homoserine, homophenylalanine, homonorleucine and homoarginine.

According some embodiments, the one amino acid is alanine, especially D-alanine.

According some embodiments, the one or more amino acids is/are not carnitine, homocystein and canavanine, neither in the L- nor in the D-configuration.

According to some embodiments, the amount of the one or more amino acids in the medicament or pharmaceutical composition is in the range from about 1:10 to about 10:1 w/w of the amount of 4-PB, each or total.

According to some embodiments, the amount of the one or more amino acids in the medicament or pharmaceutical composition is in the range from about 1:5 to about 5:1 w/w of the amount of 4-PB, each or total.

According to some embodiments, the amount of the one or more amino acids in the medicament or pharmaceutical composition is in the range from about 1:2 to about 2:1 w/w of the amount of 4-PB, each or total.

According to some embodiments, the amount of the one or more amino acids in the medicament or pharmaceutical composition is about 1:1 w/w of the amount of 4-PB, each or total.

According to some embodiments, the medicament or pharmaceutical composition according to the invention is administered one or more times a day, such as, e.g., two times, three times, four times or five times. According to some embodiments, the medicament or pharmaceutical composition according to the invention is administered once a day. According to some embodiments, the medicament or pharmaceutical composition according to the invention is administered twice a day. According to some embodiments, the medicament or pharmaceutical composition according to the invention is administered once in the morning and once in the evening.

According to some embodiments, the medicament or pharmaceutical composition according to the invention is administered two times or more a day, such as, e.g., three times, four times or five times, wherein the administrations are 2 to 14 hours apart, such as, e.g., 4, 6, 8, 10 or 12 hours apart. According to specific embodiments, the medicament or pharmaceutical composition according to the invention is administered twice a day, wherein the administrations are 8 to 12 hours apart.

According to some embodiments, the pharmaceutical composition for use according to the invention is in form of a sustained release formulation, preferably is in form of an oral sustained release formulation.

By sustained release of the active ingredient is to be understood especially a rate of release of the active ingredient during a period of about 6 to 12 or up to 24 hours.

According to some embodiments, the medicament or pharmaceutical composition further comprises at least one other therapeutic agent effective in the treatment of Alzheimer's disease.

According to some embodiments, the therapeutic agent effective in the treatment of Alzheimer's disease is a cholinesterase inhibitor. Non-limiting examples of cholinesterase inhibitor are donepezil, galantamine, rivastigmine and tacrine. Other cholinesterase inhibitors are well known to the person skilled in the art.

According to some embodiments, the therapeutic agent effective in the treatment of Alzheimer's disease is a N-methyl D-aspartate (NMDA) antagonist. A non-limiting example of a NMDA antagonist is memantine. Other NMDA antagonists are well known to the person skilled in the art.

According to some embodiments, the therapeutic agent effective in the treatment of Alzheimer's disease is a BACE 1 inhibitor. BACE 1 inhibitors are well known to the person skilled in the art, and are reviewed, for example in Guo, T., and Hobbs, D.W., Curr. Med. Chem., vol. 13, no. 15, 2006, 1811-29, and Evin, G., et al., Recent Pat CNS Drug Discov., vol. 6, no. 2" 2011, 91-106, which are hereby incorporated by reference and form part of the disclosure. Non-limiting examples of BACE 1 inhibitors are KMI-420, KMI-429, KMI-358, KMI-370, TAK-070, and GSK188909.

According to some embodiments, the therapeutic agent effective in the treatment of Alzheimer's disease is selected from donepezil, galantamine, rivastigmine, tacrine, memantine, KMI-420, KMI-429, KMI-358, KMI-370, TAK-070, and GSK188909.

According to specific embodiments, the therapeutic agent effective in the treatment of Alzheimer's disease is donepezil.

According to specific embodiments, the therapeutic agent effective in the treatment of Alzheimer's disease is galantamine.

According to specific embodiments, the therapeutic agent effective in the treatment of Alzheimer's disease is rivastigmine.

According to specific embodiments, the therapeutic agent effective in the treatment of Alzheimer's disease is tacrine.

According to specific embodiments, the therapeutic agent effective in the treatment of Alzheimer's disease is memantine.

According to specific embodiments, the therapeutic agent effective in the treatment of Alzheimer's disease is selected from KMI-420, KMI-429, KMI-358, KMI-370, TAK-070, and GSK188909.

According to some embodiments, the medicament or pharmaceutical composition comprises the at least one other therapeutic agent effective in the treatment of Alzheimer's disease in form of a salt or co-crystal of 4-PB with the therapeutic agent effective in the treatment of Alzheimer's disease.

For a definition of co-crystal reference is made to the preceding definition given above.

According to some embodiments, the therapeutic agent effective in the treatment of Alzheimer's disease which is forming a salt or co-crystal with 4-PB (as free acid) is selected from donepezil, galantamine, rivastigmine, tacrine, and memantine.

According to very specific embodiments, the therapeutic agent effective in the treatment of Alzheimer's disease which is forming a salt or co-crystal with 4-PB (as free acid) is donepezil.

According to specific embodiments, the therapeutic agent effective in the treatment of Alzheimer's disease which is forming a salt or co-crystal with 4-PB (as free acid) is galantamine.

According to specific embodiments, the therapeutic agent effective in the treatment of Alzheimer's disease which is forming a salt or co-crystal with 4-PB (as free acid) is rivastigmine.

According to specific embodiments, the therapeutic agent effective in the treatment of Alzheimer's disease which is forming a salt or co-crystal with 4-PB (as free acid) is tacrine.

According to specific embodiments, the therapeutic agent effective in the treatment of Alzheimer's disease which is forming a salt or co-crystal with 4-PB (as free acid) is memantine.

The present invention further provides kits for use in the treatment of Alzheimer's disease. According to the invention, the kits comprise at least one (first) container which contains a pharmaceutical formulation comprising 4-PB in an amount of at most about 750 mg. Suitably, 4-PB is present in the formulation together with one or more pharmaceutically acceptable carriers, diluents and/or excipients. For further details on pharmaceutically acceptable carriers, diluents and excipients reference is made to above description.

According to some embodiments, the amount of 4-PB comprised by the pharmaceutical formulation is at most about 700 mg, at most about 650 mg, at most about 600 mg, at most about 550 mg, at most about 500 mg, at most about 450 mg, at most about 400 mg, at most about 350 mg, at most about 300 mg, at most about 250 mg, at most about 200 mg, at most about 150 mg, at most about 100 mg, at most about 75 mg, at most about 50 mg or at most about 25 mg.

According to some embodiments, the amount of 4-PB comprised by the pharmaceutical formulation is at most about 100 mg

According to some embodiments, the amount of 4-PB comprised by the pharmaceutical formulation is at most about 250 mg.

According to some embodiments, the amount of 4-PB comprised by the pharmaceutical formulation is at most about 500 mg.

According to some embodiments, the amount of 4-PB comprised by the pharmaceutical formulation is at least about 25 mg, at least about 50 mg, at least about 75 mg, at least about 100 mg, at least about 150 mg, at least about 200 mg, at least about 250 mg, at least about 300 mg, at least about 350, at least about 400 mg, at least about 450 mg, at least about 500 mg, at least about 550 mg, at least about 600 mg, at least about 650 mg or at least about 700 mg.

According to some embodiments, the amount of 4-PB comprised by the pharmaceutical formulation is at least about 25 mg.

According to some embodiments, the amount of 4-PB comprised by the pharmaceutical formulation is at least about 50 mg.

According to some embodiments, the amount of 4-PB comprised by the pharmaceutical formulation is at least about 75 mg.

According to some embodiments, the amount of 4-PB comprised by the pharmaceutical formulation is at least about 100 mg.

According to some embodiments, the amount of 4-PB comprised by the pharmaceutical formulation is at least about 250 mg.

According to some embodiments, the amount of 4-PB comprised by the pharmaceutical formulation is at least about 500 mg.

According to some embodiments, the amount of 4-PB comprised by the pharmaceutical formulation is in the range from about 25 mg to about 750 mg, from about 50 mg to about 750 mg, from about 75 mg to about 750, about 100 mg to about 750 mg, from about 150 mg to about 750 mg, from about 200 mg to about 750 mg, from about 250 mg to about 750 mg, from about 300 mg to about 750 mg, from about 350 mg to about 750 mg, from about 400 mg to about 750 mg, from about 450 mg to about 750 mg, from about 500 mg to about 750 mg, from about 550 mg to about 750 mg, from about 600 mg to about 750 mg, from about 650 to about 750 mg or from about 700 mg to about 750 mg.

According to some embodiments, the amount of 4-PB comprised by the pharmaceutical formulation is in the range from about 25 mg to about 500 mg, from about 50 mg to about 500 mg, from about 75 mg to about 500 mg, from about 100 to about 500 mg, from about 150 to about 500 mg, from about 200 mg to about 500 mg, from about 250 mg to about 500 mg, from about 300 mg to about 500 mg, from about 350 mg to about 500 mg, from about 400 mg to about 500 mg or from about 450 to about 500 mg.

According to some embodiments, the amount of 4-PB comprised by the pharmaceutical formulation is in the range from about 25 mg to about 250 mg, from about 50 mg to about 250 mg, from about 75 mg to about 250 mg, from about 100 to about 250 mg, from about 125 mg to about 250 mg, from about 150 to about 250 mg, from about 175 mg to about 250 mg or from about 200 mg to about 250 mg.

According to some embodiments, the amount of 4-PB comprised by the pharmaceutical formulation is in the range from about 25 mg to about 100 mg, from about 50 mg to about 100 mg, from about 55 mg to about 100 mg, from about 60 mg to about 100 mg, from about 65 mg to about 100 mg, from about 70 mg to about 100 mg, from about 75 mg to about 100 mg, from about 80 mg to about 100 mg, from about 85 mg to about 100 mg, from about 90 mg to about 100 mg or from about 95 to about 100 mg.

According to some embodiments, the pharmaceutical formulation comprises 4-PB in the form of a pharmaceutically acceptable salt, co-crystal, polymorph, hydrate, solvate or pro-drug. For further details on pharmaceutically acceptable salts, co-crystals, polymorphs, hydrates, solvates and pro-drugs of 4-PB reference is made to above description. According to some embodiments, the 4-PB salt is an alkali metal salt selected from the group of lithium, sodium, potassium, calcium, magnesium, aluminium and cesium.

According to some embodiments, the 4-PB salt is the sodium salt, sodium 4-phenylbutyrate.

According to some embodiments, the kits further comprise at least a further (such as, e.g., a second, third, fourth, etc.) container which contains a pharmaceutical formulation comprising one or more amino acids (such as, e.g. two, three, four, five, six, seven, eight, nine or ten amino acids). Suitably, the one or more amino acids is/are present in the pharmaceutical formulation together with one or more pharmaceutically acceptable carriers, diluents and/or excipients. For further details on pharmaceutically acceptable carriers, diluents and excipients reference is made to above description.

According to some embodiments, the one or more amino acids is/are L-amino acid(s) or D-amino acid(s), or a combination thereof.

According to specific embodiments, the one or more amino acids is/ are in the D-configuration.

According to some embodiments, the one or more amino acids is/are selected from nonpolar, polar, acidic or basic amino acids.

According to some embodiments, the one or more amino acids is/are selected from aliphatic, sulfur-containing, aromatic or neutral amino acids.

According some embodiments, the one or more amino acids is/are selected from alanine, valine, leucine, isoleucine, proline, methionine, phenylalanine and tryptophane.

According some embodiments, the one or more amino acids is/are selected from serine, threonine, asparagine, glutamine, cysteine and tyrosine.

According some embodiments, the one or more amino acids is/are selected from aspartic acid and glutamic acid.

According some embodiments, the one or more amino acids is/are selected from lysine, arginine and histidine.

According some embodiments, the one or more amino acids is/are selected from alanine, valine, Leucine and isoleucine.

According some embodiments, the one or more amino acids is/are selected from cysteine and methionine.

According some embodiments, the one or more amino acid is/are selected from phenylalanine, tyrosine and tryptophane.

According some embodiments, the one or more amino acid is/are selected from serine, threonine, asparagine and glutamic acid.

According some embodiments, the one or more amino acid is/are selected from selenocysteine, selenomethionine, norleucine, norvaline, ornithine, penicillamine, hydroxyproline, homoserine, homophenylalanine, homoleucine, homonorleucine and homoarginine,

According some embodiments, the one or more amino acid is selected from alanine, arginine, aspargine, aspartic acid, cysteine, glutamic acid, glutamine, histidine, Isoleucine, leucine, lysine, methionine, phenylalanine, proline, serine, threonine, tryptophane, tyrosine, valine, selenocysteine, selenomethionine, norleucine, norvaline, ornithine, penicillamine, hydroxyproline, homoserine, homophenylalanine, homonorleucine and homoarginine.

According some embodiments, the one amino acid is alanine, especially D-alanine.

According some embodiments, the one or more amino acids is/are not carnitine, homocystein and canavanine, neither in the L- nor in the D-configuration.

According to some embodiments, the amount of the one or more amino acids in the kit is in the range from about 1:10 to about 10:1 w/w of the amount of 4-PB, each or total.

According to some embodiments, the amount of the one or more amino acids in the kit is in the range from about 1:5 to about 5:1 w/w of the amount of 4-PB, each or total.

According to some embodiments, the amount of the one or more amino acids in the kit is in the range from about 1:2 to about 2:1 w/w of the amount of 4-PB, each or total.

According to some embodiments, the amount of the one or more amino acids in the kit is about 1:1 w/w of the amount of 4-PB, each or total.

Where more than one amino acid is comprised by the kit, each amino acid may be presented within an individual pharmaceutical formulation contained in a separate container.

According to some embodiments, the kits further comprise at least a further (such as, e.g., a second, third, fourth, etc.) container which contains a pharmaceutical formulation comprising at least one other therapeutic agent effective in the treatment of Alzheimer's disease. Suitably, the at least one other therapeutic agent effective in the treatment of Alzheimer's disease is present in the pharmaceutical formulation together with one or more pharmaceutically acceptable carriers, diluents and/or excipients. For further details on pharmaceutically acceptable carriers, diluents and excipients reference is made to above description.

According to some embodiments, the therapeutic agent effective in the treatment of Alzheimer's disease is a cholinesterase inhibitor. Non-limiting examples of cholinesterase inhibitor are donepezil, galantamine, rivastigmine and tacrine. Other cholinesterase inhibitors are well known to the person skilled in the art.

According to some embodiments, the therapeutic agent effective in the treatment of Alzheimer's disease is a N-methyl D-aspartate (NMDA) antagonist. A non-limiting example of a NMDA antagonist is memantine. Other NMDA antagonists are well known to the person skilled in the art.

According to some embodiments, the therapeutic agent effective in the treatment of Alzheimer's disease is a BACE 1 inhibitor. BACE 1 inhibitors are well known to the person skilled in the art, and are reviewed, for example in Guo, T., and Hobbs, D.W., Curr. Med. Chem., vol. 13, no. 15, 2006, 1811-29, and Evin, G., et al., Recent Pat CNS Drug Discov., vol. 6, no. 2,, 2011, 91-106, which are hereby incorporated by reference and form part of the disclosure. Non-limiting examples of BACE 1 inhibitors are KMI-420, KMI-429, KMI-358, KMI-370, TAK-070, and GSK188909.

According to some embodiments, the therapeutic agent effective in the treatment of Alzheimer's disease is selected from donepezil, galantamine, rivastigmine, tacrine, memantine, KMI-420, KMI-429, KMI-358, KMI-370, TAK-070, and GSK188909.

According to specific embodiments, the therapeutic agent effective in the treatment of Alzheimer's disease is donepezil.

According to specific embodiments, the therapeutic agent effective in the treatment of Alzheimer's disease is galantamine.

According to specific embodiments, the therapeutic agent effective in the treatment of Alzheimer's disease is rivastigmine.

According to specific embodiments, the therapeutic agent effective in the treatment of Alzheimer's disease is tacrine.

According to specific embodiments, the therapeutic agent effective in the treatment of Alzheimer's disease is memantine.

According to specific embodiments, the therapeutic agent effective in the treatment of Alzheimer's disease is selected from KMI-420, KMI-429, KMI-358, KMI-370, TAK-070, and GSK188909.

Where more than one therapeutic agent effective in the treatment of Alzheimer's disease is comprised by the kit, each therapeutic agent may be presented within an individual pharmaceutical formulation contained in a separate container.

According to some embodiments, the kit comprises the at least one other therapeutic agent effective in the treatment of Alzheimer's disease in form of a salt or co-crystal of 4-PB with the therapeutic agent effective in the treatment of Alzheimer's disease.

For a definition of co-crystal reference is made to the preceding definition given above.

According to some embodiments, the therapeutic agent effective in the treatment of Alzheimer's disease which is forming a salt or co-crystal with 4-PB (as free acid) is selected from donepezil, galantamine, rivastigmine, tacrine, and memantine.

According to very specific embodiments, the therapeutic agent effective in the treatment of Alzheimer's disease which is forming a salt or co-crystal with 4-PB (as free acid) is donepezil.

According to specific embodiments, the therapeutic agent effective in the treatment of Alzheimer's disease which is forming a salt or co-crystal with 4-PB (as free acid) is galantamine.

According to specific embodiments, the therapeutic agent effective in the treatment of Alzheimer's disease which is forming a salt or co-crystal with 4-PB (as free acid) is rivastigmine.

According to specific embodiments, the therapeutic agent effective in the treatment of Alzheimer's disease which is forming a salt or co-crystal with 4-PB (as free acid) is tacrine.

According to specific embodiments, the therapeutic agent effective in the treatment of Alzheimer's disease which is forming a salt or co-crystal with 4-PB (as free acid) is memantine.

According to some embodiments, also the medicaments, pharmaceutical compositions and pharmaceutical formulations as disclosed herein in the context of other aspects of the present invention may form part of a kit according to the invention, and as such may be comprised within one or more containers of such kit.

The present invention further provides a salt or co-crystal of 4-PB with a therapeutic agent effective in the treatment of Alzheimer's disease.

For a definition of co-crystal reference is made to the preceding definition given above.

Such a salt or c-crystal of 4-PB (especially as free acid) with a therapeutic agent effective in the treatment of Alzheimer's disease ameliorates and effectively treats the symptoms associated with Alzheimer's disease, especially if used at a low dosis.

According to some embodiments, the therapeutic agent effective in the treatment of Alzheimer's disease which is forming a salt or co-crystal with 4-PB is selected from donepezil, galantamine, rivastigmine, tacrine, and memantine.

According to some embodiments, the therapeutic agent effective in the treatment of Alzheimer's disease which is forming a salt with 4-PB (as free acid) is selected from donepezil, galantamine, rivastigmine, tacrine, and memantine.

According to specific embodiments, the salt of 4-PB with a therapeutic agent effective in the treatment of Alzheimer's disease is a salt of 4-PB as free acid with donepezil.

According to specific embodiments, the salt of 4-PB with a therapeutic agent effective in the treatment of Alzheimer's disease is a salt of 4-PB as free acid with galantamine.

According to specific embodiments, the salt of 4-PB with a therapeutic agent effective in the treatment of Alzheimer's disease is a salt of 4-PB as free acid with rivastigmine.

According to specific embodiments, the salt of 4-PB with a therapeutic agent effective in the treatment of Alzheimer's disease is a salt of 4-PB as free acid with tacrine.

According to specific embodiments, the salt of 4-PB with a therapeutic agent effective in the treatment of Alzheimer's disease is a salt of 4-PB as free acid with memantine.

According to some embodiments, the therapeutic agent effective in the treatment of Alzheimer's disease which is forming a co-crystal with 4-PB (as free acid or salt) is selected from donepezil, galantamine, rivastigmine, tacrine, and memantine.

According to specific embodiments, the co-crystal of 4-PB with a therapeutic agent effective in the treatment of Alzheimer's disease is a co-crystal of 4-PB (as free acid or salt) with donepezil.

According to specific embodiments, the co-crystal of 4-PB with a therapeutic agent effective in the treatment of Alzheimer's disease is a co-crystal of 4-PB (as free acid or salt) with galantamine.

According to specific embodiments, the co-crystal of 4-PB with a therapeutic agent effective in the treatment of Alzheimer's disease is a co-crystal of 4-PB (as free acid or salt) with rivastigmine.

According to specific embodiments, the co-crystal of 4-PB with a therapeutic agent effective in the treatment of Alzheimer's disease is a co-crystal of 4-PB (as free acid or salt) with tacrine.

According to specific embodiments, the co-crystal of 4-PB with a therapeutic agent effective in the treatment of Alzheimer's disease is a co-crystal of 4-PB (as free acid or salt) with memantine.

The present invention further provides a medicament or pharmaceutical composition comprising a salt or co-crystal of 4-PB with a therapeutic agent effective in the treatment of Alzheimer's disease according to the invention and optionally one or more pharmaceutically acceptable carriers, diluents and/or excipients.

According to specific embodiments, the medicament or pharmaceutical composition is for use in the treatment of Alzheimer' disease.

According to other specific embodiments, the medicament or pharmaceutical composition is comprising the salt or co-crystal of 4-PB with a therapeutic agent effective in the treatment of Alzheimer's disease according to the invention in an amount of at most about 750mg, preferably at most about 500mg or 250mg of 4-PB in the salt or co-crystal.

The present invention further provides the salt or co-crystal of 4-PB with a therapeutic agent effective in the treatment of Alzheimer's disease according to the invention for use in the treatment of Alzheimer' disease.

It is again explicitly intended that the teaching of this invention covers any combination of the above described embodiments.

The present invention is illustrated below with the help of the following figures and examples. These illustrations are given solely by way of example and do not limit the invention.

### Examples

### Example 1: Production of slow release tablet with 250 mg of sodium 4-phenylbutyrate

A mixture of 6,000.0 g of sodium 4-phenylbutyrate from Triple Crown America, Inc., 6,280.0 g of lactosum monohydriculum, 3,500.0 g of Methocel K100 M Preium (Prochem), and 750.0 g of Avicel PH 102 (Select Chemie) is wettened with 4,000.0 g of aqua purificata (water purified by inversion osmosis) and dried in cold air during 10 hours with air of 40°C. A mixture of 240.0 g of talcum and 30.0 g of magnesium stearate is admixed during 20 minutes and the mixture is pressed into tablets of 0.70 g each, a thickness of about 6.8 mm and with a hardness of 90 Newton. Yield: 24,000 tablet cores.

The mixing is carried out with a Diosna Mixer, the drying in a Lükon drying cabinet, the sieving with a Köhler & Bosshard sieving machine, and the tablet pressing with a Korsch tablet press EK 11.

The cores are provided with a film coating for example by using a colloidal dispersion containing 7,850 g of isopropylalcohol, 3,360 g of Eudragit L 12.5, 66 g of dibutyl phthalat, 18.0 g of Miglyol 812, and 56 g of polyethylenglycol PEG 400. The suspension is sprayed at 3.5 atü and 25° C onto the 24,000 cores. The film-coated tablets are dried in a circulating air drying cabinet for at least 4 hours at 35°C.

## Claims

1. 4-phenylbutyric acid (4-PB) for use in the treatment of Alzheimer's disease, wherein 4-PB is administered to a subject in need thereof in a daily amount of at most about 750 mg.

2. 4-phenylbutyric acid for use according to claim 1, wherein the daily amount of 4-PB to be administered it at most about 700 mg, at most about 650 mg, at most about 600 mg, at most about 550 mg, at most about 500 mg, at most about 450 mg, at most about 400 mg, at most about 350 mg, at most about 300 mg, at most about 250 mg, at most about 200 mg, at most about 150 mg, at most about 100 mg, at most about 75 mg, at most about 50 mg or at most about 25 mg.

3. 4-phenylbutyric acid for use according to claim 1 or 2, wherein the daily amount of 4-PB to be administered is at most about 250 mg or 500 mg,
and/or
wherein the daily amount of 4-PB to be administered is at least about 25 mg or 50 mg.

4. 4-phenylbutryic acid for use according to any one of claims 1 to 3, wherein 4-PB is in the form of a pharmaceutically acceptable salt, co-crystal, polymorph, hydrate, solvate or pro-drug.

5. 4-phenylbutryic acid for use according to any one of claims 1 to 4, wherein 4-PB is administered in combination with one or more amino acids, such as, e.g., D-amino acid(s).

6. 4-phenylbutryic acid for use according to any one of claims 1 to 5, wherein 4-PB is administered in combination with at least another treatment of Alzheimer's disease.

7. Pharmaceutical composition for use in the treatment of Alzheimer' disease, comprising 4-phenylbutyric acid in an amount of at most about 750mg and one or more pharmaceutically acceptable carriers, diluents and/or excipients.

8. The pharmaceutical composition for use according to claim 7, wherein the amount of 4-PB it at most about 700 mg, at most about 650 mg, at most about 600 mg, at most about 550 mg, at most about 500 mg, at most about 450 mg, at most about 400 mg, at most about 350 mg, at most about 300 mg, at most about 250 mg, at most about 200 mg, at most about 150 mg, at most about 100 mg, at most about 75 mg, at most about 50 mg or at most about 25 mg.

9. The pharmaceutical composition for use according to claim 7 or 8, wherein the amount of 4-PB is at most about 250 mg or 500 mg,
and/or
wherein the amount of 4-PB is at least about 25 mg or 50 mg.

10. The pharmaceutical composition for use according to any one of claims 7 to 9, wherein 4-PB is in the form of a pharmaceutically acceptable salt, co-crystal, polymorph, hydrate, solvate or pro-drug.

11. The pharmaceutical composition for use according to any one of claims 7 to 10, further comprising one or more amino acid(s), such as, e.g., D-amino acid(s).

12. The pharmaceutical composition for use according to any one of claims 7 to 11, further comprising at least one other therapeutic agent effective in the treatment of Alzheimer's disease.

13. Kit for use in the treatment of Alzheimer's disease, comprising at least one container which contains a pharmaceutical formulation comprising 4-phenylbutyric acid in an amount of at most about 750 mg, and, optionally, at least a second container which contains a pharmaceutical formulation comprising one or more amino acids, such as, e.g. D-amino acid(s), and/or at least one other therapeutic agent effective in the treatment of Alzheimer's disease.

14. Salt or co-crystal of 4-PB with a therapeutic agent effective in the treatment of Alzheimer's disease, preferably with the therapeutic agent effective in the treatment of Alzheimer's disease being selected from donepezil, galantamine, rivastigmine, tacrine, and memantine.

15. Salt or co-crystal of 4-PB according to claim 14 selected from
- a salt of 4-PB as free acid with donepezil,
- a salt of 4-PB as free acid with galantamine,
- a salt of 4-PB as free acid with rivastigmine,
- salt of 4-PB as free acid with tacrine,
- a salt of 4-PB as free acid with memantine,
- a co-crystal of 4-PB (as free acid or salt) with donepezil,
- a co-crystal of 4-PB (as free acid or salt) with galantamine,
- a co-crystal of 4-PB (as free acid or salt) with rivastigmine,
- a co-crystal of 4-PB (as free acid or salt) with tacrine, or
- a co-crystal of 4-PB (as free acid or salt) with memantine.
